# EUROPEAN PATENT SPECIFICATION

(11) **EP 1 901 697 B1**
(45) Date of publication and mention of the grant of the patent: **14.06.2017**
(21) Application number: 05769043.0
(22) Date of filing: 30.06.2005
(51) Int. Cl.: A61K 6/00

(54) **Herbal formulation for the prevention and mangement of diabetes mellitus and diabetic micro-vascular complications**
Pflanzliche Formulierung zur Prävention und Behandlung von Diabetes mellitus und diabetischen mikrovaskulären Komplikationen
Formulation a base de plantes destinee a prevenir ou a gerer le diabete sucre et les complications microvasculaires diabetiques

(30) Priority: 30.05.2005 IN DE13972005
(43) Date of publication of application: 26.03.2008
(73) Proprietor: Gill, Harinder Singh, Bathinda PU (IN); Gill, Gurpreet Singh, Bathinda PU (IN)
(72) Inventor: DUBEY, Govind, Prasad, Varanasi 221005 (IN); AGARWAL, Aruna, Varanasi 221005 (IN); VYAS, Neers, New Delhi (IN); RAJAMANICKAM, Victor, G, Thanjavur (IN)
(74) Representative: Lavoix
(86) International application number: PCT/IN2005/000223
(87) International publication number: WO 2006/129325

(56) References cited:
- US-A1- 2005 031 718
- DHANABAL S.P. ET AL.: 'The hypoglycemic activity of Coccinia indica Wight & Arn. and its influence on certain biochemical parameters' INDIAN J. PHARMACOL. vol. 36, no. 4, August 2004, pages 249 - 250, XP003008862
- PATENT ABSTRACTS OF JAPAN & JP 2002 267655 A (MORISHITA JINTAN KK) 18 September 2002
- SANDHU A S ET AL: "Potential of Ayurvedic herbs in the treatment of diabetes mellitus", MEDICINAL & AROMATIC PLANTS ABSTRACTS, SCIENTIFIC PUBLISHERS, SCIENTIFIC PUBLISHERS, NEW DELHI - INDIA, vol. 27, no. 4, 1 August 2005 (2005-08-01) , XP018012371, ISSN: 0250-4367
- SASTRY ET AL: "Novelistic approach to the management of diabetes through polymolecular herbal drugs", MEDICINAL & AROMATIC PLANTS ABSTRACTS, SCIENTIFIC PUBLISHERS, NEW DELHI - INDIA, vol. 26, no. 1, 1 February 2004 (2004-02-01), XP018001980,
- DATABASE WPI Week 200349, Derwent Publications Ltd., London, GB; AN 2003-516823 & JP 2003 063974 A (NIPPON BIO KK) 05 March 2003

## Description

### FIELD OF INTENTION:

This invention relates to a herbal formulation according to claim 1 for the prevention of Diabetis Mellitus and Diabetic Micro-Vascular Complications. The herbal formulation according to claim 1 may also be advantageously employed for endothelial dysfunction.

### BACKGROUND OF INVENTION:

Pre-diabetes is a serious medical condition which is modifiable. Recently, Diabetes Prevention Program Study has shown that patients with pre-diabetes can prevent the development of type II diabetes by a proper treatment, which can even reduce the blood glucose levels to the normal range. A number of studies have indicated that patients with pre-diabetes can prevent or delay the development of type II diabetes by up to 58 percent through changes to their lifestyle. Normal blood glucose is bellow 140 mg/dl two hours after the drink. In pre-diabetes the 2-hour blood glucose is 140 to199 mg/dl and if the 2-hour blood glucose rises to 200 mg/dl or above, a patient has diabetes.

For adults who are younger than 45 and over weight, along with other risk factors for diabetes or pre-diabetes like high blood pressure, low HDL cholesterol and high triglycerides, a family history of diabetes, a history of gestational diabetes etc are at high risk for diabetes. In fact, symptoms such as unusual thirst, frequent desire to urinate, blurred vision or a feeling of being tired most of the time for no apparent reason are often not recognized by patients. Such patients are more susceptible to coronary and peripheral artery disease and also show abnormal lipid profile.

Diabetes Control and Complications Trial (DCCT) has provided considerable information regarding the molecular mechanism, prevention and control of diabetic complications among those individuals who are suffering from different type of diabetes.

Diabetes Control and Complications Trial emerged after a comprehensive study to establish the scientific connection between the level of diabetic control and the risk of angiopathic complications in mass. The diabetic complications are mainly based on uncontrolled management of diabetes mellitus. Microangiopathic changes results in microalbuminuria, degenerative changes in micro vessels, abnormality into lipid metabolism etc. ultimately leads to gross renal, cardiac or retinopathic consequences.

Recent epidemiological studies have shown that due to varying reasons the incidence of diabetes mellitus is increasing. In one of the WHO reports the Indians are more prone to develop hypertriglyceridemia and diabetes mellitus in comparison to western countries. Diabetes is world wide in distribution and the incidence of both types of primary diabetes i.e. insulin dependent diabetes (IDDM) and non-insulin dependent diabetes (NIDDM) is increasing due to urban way of life style. However, the prevalence of both type varies considerably in different parts of the world. This seems to be due to the differences in both genetic and environmental factors. Different epidemiological studies have demonstrated that prevalence of non-insulin dependent diabetes is many times more than the insulin dependent diabetes. Though the precise etiology of diabetes is still uncertain, the environmental factors interact with a genetic susceptibility to determine which of those with a genetic predisposition actually develop the clinical syndrome and the timings of its onset. In addition to genetic, autoimmune factor also play a vital role in the onset of diabetes.

Referring to the application for endothelial dysfunction, the use of an extract of salacia oblonga is not known. However, Patricia M. Heacock et. al. has disclosed in the journal of American Dietetie Association, January 2005, the use of salacia oblonga extract as a herbal X-glucosidase inhibitor are post prandial glycemic, insulinemic in patients. Results on use of such an extract reflected that only a dosage of 1000 mg salacia oblonga reduced the plasma, glucose and serum insulin incremental areas. The other doses of S oblonga extract did not impact glycemia or insulinemia.
Further, such a paper did not teach the application of S oblonga extract with respect to endothetial dysfunction.

### OBJECTS OF INVENTION:

An object of this invention is to propose a herbal formulation according to claim 1 for the prevention of Diatetic Mellitus and other complications caused by Diabetes and for endothelial dysfunction.

Another object of this invention is to propose a herbal formulation according to claim 1 which would delay or stop the use of insulinin patients suffering from type II diabetes.

Further object of this invention is to propose a herbal formulation according to claim 1 which minimizes endothelial dysfunction in pre-diabetic and also diabetic cases.

Still further object of this invention is to propose a herbal formulation according to claim 1 which regulates the abnormal lipo protein metabolism among the diabetics patients.

### STATEMENT OF THE INVENTION:

According to this invention there is provided a herbal formulation according to claim 1 for use in the prevention of endothelial dysfunction, and Diabetes Mellitus and Diabetic Micro-Vascular Complications comprising Salacia chinensis atleast one other active constituent selected from Coccinia indica and Hippophae rhamnoides and optionally additives in trace amounts.

Further, according to this invention there is also provided a process of making a herbal formulation according to claim 1 comprising mixing an organic extract of Salacia chinensis and atleast one of the active constituent in selected from Hippophae-rhamnoides Coccinia indica and optionally additives in trace amounts.

### DETAILED DESCRIPTION OF THE PRESENT INVENTION:

Coccinia indica is a creeping plant found in entire India particularly in the Tribal locality of Vindhya and Satpura hills. It has three species namely Coccinia grandis, Coccinia indica and Coccinia cordifolia. The fruits of the plant is essentials used for a vegetable. It contain β-sitosterol and taraxerol from fruits; seeds are rich in essential fatty acid like palmitic acid, oleic acid and linoleic acids. The use of Coccinia indica as a herbal preparation for the applications described herein Is not hitherto known.

### Hippophae rhamnoides:

Hippophae rhamnoides belongs to the family of 'Elaegnaceae', is a small genus of shrubs and trees and in native of temperate region. It contains vitamins such as carotene, flavonoides, folic acid, fatty acids, and tannic acid. The ripe fruits contain many typy of vitamins and stem contains 5-HT also. The use of Hippophae rhamnoides as a herbal preparation for the applications described herein is not hitherto known.

### Salacia chinensis:

Salacia is a small deciduous plant found in eastern and western Peninsula, Meghalaya and in the central India particularly near the bank of Narmada valley up to the 3000 feet from sea level. The length of leaves varies from 3 to 6 inches and about 1 to 2 inches. The flowering starts in the month of Dec and Jan, and fruits appears in the month of April. The bark of root is golden colour and after cutting the root, seven wheel appears and it has a variety of colours. This plant has have a 3 sub species namely Salacia chinensis, Salacia latifolia and Salacia oblonga. It is also known as Salacia prinoides. The root is utilized by the local people for the treatment of inflammatory process and also for liver disorders and for the management of polyurea and polydyspepsia. The use of Salacia chinensis as a herbal preparation for the applications described herein is not hitherto known.

The formulation according to claim 1 of the present invention comprises the following constituents:
1. Coccinia indica (Bimbl)
2. Hippophae rhamnoides (Badriphal)
3. Salacia chinensis (Sapta chakra)
and present in the range of:

| | | |
|---|---|---|
| 1. Coccinia indica | - | 200-400 mg. |
| 2. Hippophae rhamnoides | - | 200-400 mg. |
| 3. Salacia chinensis | - | 350-550 mg. |

for every 1000 mg of said formulations.
also preferably the formulation comprises:

| | | |
|---|---|---|
| 1. Coccinia indica | - | 200-300 mg. |
| 2. Hippophae rhamnoides | - | 250-400 mg. |
| 3. Salacia chinensis | - | 400-500 mg. |

for every 1000 mg of the preparation.

The formulation may also comprise known additives such as mineral, vitamin, salts, fillers (for encapsulation) and binders, if required and present in trace amounts.

### Extraction procedure of Salacia chinensis:

Root of Salacia chinensis is used for the hydro - alcoholic extraction. Extraction was done through hot percolation method by soxhlet apparatus using water and alcohol as a solvent. Extraction was carried out continuously for 70 hrs. at boiling point of the solvent used. After 70 hrs, maintaining the same temperature solvent used in the extraction was recovered to a possible amount through single distillation. The obtained hydro-alcoholic extract was solidified to a pasty mass over thermostatic water bath at a maintained temperature as above. The yield was calculated after weighing the extracted quantity.

### Quantitative estimation of alcoholic extract of Salacia chinensis for the separation of active chemical constituents of Salacia chinensis by chromatographic procedures.

After extraction of organic extract of Salacia chinensis, a chromatographic separation was carried out by using thin layer column and high performance liquid chromatography. Various chemical compounds like leucopelargonidin, tetramer, dulcitol and gutta were separated and characterized on the basis of different results obtained by IR, UV, NMR for proton, NMR for carbon, Mass and elemental analysis.

### Extraction procedure of Hippophae rhamnoides and Coccinia Indica:

The dried fruits of Hippophae rhamnoides was extracted with hydro-alcoholic solvent (70 : 30) at the temperature of 60 to 70 continuously for 70 hrs. After a detailed chemical characterization the extracted material was taken for assessment of therapeutic potentials. The same extraction procedure was adopted to obtain the yield of the plant Coccinia indica for purpose of preparation of the formulation.

After processing the yield with column chromatography of the plants under investigation HPTLC and spectroscopic method the extract were taken for the assessment of their safety and efficacy profile.

The herbal formulation according to claim 1 of the present invention was obtained by mixing the organic extract of Salacia chinensis with the extract of Hippophae rhamnoides and Coccinia indica.

The extract of Salacia oblonga when taken twice (1000 mg in two equal doses) showed reduction in blood glucose level in comparison to control series but Salacia chinensis showed better effect on blood glucose level in comparison to salacia oblonga. Similarly, when diabetics is caused in rats using streptozotocin a far better result is obtained after 12 weeks with Salacia chinensis compared to salacia oblonga as salacia chinensis showed contant decline in blood glucose level as clearly shown in Table 1.

**Table 1:**

| **Effect of Salacia chinensis and Salacia oblonga on Blood glucose level among Streptozotocin treated rats - a comprehensive study** | | | | |
|---|---|---|---|---|
| Experimental Groups | Blood Glucose level (mg%) | | | |
| | Initial | After 7 days | After 6 weeks | After 12 weeks |
| Normal control (N=10) | 63.97 ±3.42 | 68.44 ±5.02 | 67.01 ±4.82 | 64.73 ±5.08 |
| Treated with Streptozotocin (N=10)* | 67.11 ±3.97 | 192.84 ±11.64 | 176.94 ±7.72 | 164.80 ±8.22 |
| Streptozotocin + Salacia chinensis (N=10)** | 64.72 ±6.84 | 176.01 ±6.42 | 144.52 ±5.11 | 102.98 ±4.28 |
| Streptozotocin + Salacia oblonga (N=10)*** | 67.94 ±5.83 | 182.45 ±9.25 | 161.02 ±5.85 | 123.44 ±6.02 |

| | | | | |
|---|---|---|---|---|
| **Comparison:** * vs** p>0.05 p<0.01 p<0.001 p<0.001 * vs*** p>0.05 p<0.05 p<0.001 p<0.001 **vs*** p>0.05 p<0.05 p<0.001 p<0.001 | | | | |

The results of table 2 initially, reflects that when organic extract of Salacia chinensis, Hippophae rhamnoides and Coccinia indica was given simultaneously the decrease in blood glucose level is less in animals compared to the results obtained by administrating salacia chinensis alone. Whereas, table 2 also shows that in the long run say after 3 months the combined effect of Salacia chinensis, Hippophae rhamnoides and Coccinia indica give much better results. Thus, the herbal formulation reduces the blood glucose level to a great extent both for fasting and post prandial PP. Table 2:

**Table 2:**

| **Clinical study Effect of Salacia chinensis and novel test formulation in blood glucose level among pre diabetes cases.** | | | | | | |
|---|---|---|---|---|---|---|
| Treatment groups | Blood glucose level (mg%) | | | | Comparison | |
| | Initial | | After 3 months | | Initial Vs After 3 months | Initial Vs After 3 months |
| | Fasting | PP | Fasting | PP | | |
| Control Groups (only dietary control) (N=48) | 118.45 ±6.25 | 193.74 ±8.92 | 124.29 ±6.43 | 188.72 ±9.54 | P<0.001 | P<0.05 |
| Treated Group Dietary control + Salacia chinensis (N=53) | 121.73 ±9.42 | 197.88 ±11.64 | 104.22 ±5.28 | 157.34 ±4.84 | P<0.001 | P<0.001 |
| Treated group dietary control + test formulation organic extract of Salacia chinensis & Hippophae Rhamnoides & Coccinia indica (N=61) | 119.48 ±8.64 | 201.94 ±10.40 | 98.02 ±4.62 | 145.35 ±6.25 | P<0.001 | P<0.001 |

Table 3 shows that the combined effect of the three ingredients of the herbal formulation gives a better result in rats which are treated with streptozotocin. After a duration of 12 weeks it is seen that blood glucose level decreases when streptozotocin treated rats are given the dose of herbal formulation comprising Salacia chinensis, Hippophae rhamnoides and Coccinia indica.

**Table 3:**

| **Effect of organic extract of Salacia chinensis, Hippophae rhamnoides and Coccinia indica on blood glucose level in Streptozotocin treated experimental rats.** | | | |
|---|---|---|---|
| Experimental group | Blood Glucose level (mg/%) | | |
| | Initial | After 6 weeks | After 12 weeks |
| Normal control (N=10)* | 63.97 ±3.42 | 67.01 ±4.82 | 64.73 ±5.08 |
| Streptozotocin treated (N=10)** | 67.11 ±3.92 | 176.94 ±7.72 | 164.80 ±8.22 |
| Streptozotocin + Salacia chinensis & Hippophae rhamnoides & Coccinia indica (N=10)*** | 61.45 ±4.52 | 158.84 ±9.02 | 94.02 ±5.11 |

| | | | |
|---|---|---|---|
| Comparison: *vs** p>0.05 p>0.001 p<0.001 *vs*** p>0.05 p>0.001 p<0.001 **vs*** p<0.05 p>0.001 p<0.001 | | | |

The marked increase in the level of C-reaction protein (CRP), homocystin and interleukin 6 in diabetes mellitus type II causes endothelial dysfunction.

This endothelial dysfunction is regulated to great extent by the use of herbal formulation (containing organic extract of Salacia chinensis, Hippophae rhamnoides & Coccinia indica), as shown in Table 4:

**Table 4:**

| **Endothelial dysfunction and its regulation by a herbal formulation among, diabetes mellitus type II cases** | | | | | | | |
|---|---|---|---|---|---|---|---|
| parameters | Group | Initial | After 6 week | After 12 weeks | After 18 weeks | After 24 weeks | After 30 week |
| CRP (mg/lit.) | Test drug treated | 3.22 ±1.72 | 2.05 ±1.82 | 1.35 ±0.85 | 1.35 ±0.38 | 1.48 ±0.47 | 1.28 ±0.35 |
| | Anti Diabetic agent (Control) | 3.28 ±1.02 | 3.10 ±0.98 | 2.98 ±0.80 | 3.06 ±0.75 | 2.85 ±0.68 | 2.82 ±0.64 |
| Homocysteine (µmol/lit.) | Test drug treated | 21.85 ±5.82 | 16.42 ±3.85 | 17.2 ±4.28 | 12.80 ±4.75 | 0.5 ±3.9 | 8.63 ±4.6 |
| | Anti diabetic agent (Control) | 23.45 ±2.73 | 23.21 ±3.01 | 22.78 ±3.04 | 23.11 ±2.94 | 22.98 ±2.72 | 22.94 ±2.44 |
| Inter-leukin6 (pg/ml) | Test drug treated | 3.45 ±1.75 | 2.24 ±1.28 | 1.92 ±0.98 | 1.70 ±0.75 | 1.62 ±0.78 | 1.24 ±0.62 |
| | Anti diabetic Agent (Control) | 3.52 ±1.03 | 3.45 ±0.75 | 3.28 ±0.92 | 3.46 ±0.84 | 3.18 ±0.65 | 3.08 ±0.58 |

The organic extract of Salacia chinensis alongwith Hippophae rhamnoides and Coccinia indica was administrated to prediabetic cases which showed significant decrease in low density lipoprotein, cholesterol and HDLC compared to the organic extract of Salacia chinensis alone. Table 5 & 5(a) clearly reflects that the combined effect of herbal formulation of the present invention gives much better results.

**Table 5:**

| **Effect of Salacia chinensis and Novel Test Formulation in the regulation of LDL-c and HDL-c** | | | | | | |
|---|---|---|---|---|---|---|
| **Treatment goup** | **Low density Lipoprotein (mg/dl)** | | **Comparison Initial vs After 3 months** | **HDL Cholesterol (mg/dl)** | | **Comparison Initial vs After 3 months** |
| | **Initial** | **After 3 months** | | **Initial** | **After 3 months** | |
| Control group (only dietary control) (N=48) | 123.84 ±6.23 | 118.56 ±5.95 | P<0.001 | 47.95 ±2.82 | 45.88 ±3.02 | P<0.01 |
| Treated Salacia chinensis along with dietary control (N=53) | 125.65 ±7.01 | 110.85 ±5.35 | P<0.001 | 46.28 ±2.11 | 48.01 ±3.11 | P<0.01 |
| Treated withTest formulation along with dietary control (N+61) | 126.38 ±6.82 | 93.82 ±6.02 | P<0.001 | 44.25 ±2.06 | 50.23 ±4.03 | P<0.001 |

**Table 5a:**

| **Effect of Salacia chinensis and Novel Test Formulation in the regulation of Total cholesterol and triglycerides** | | | | | | |
|---|---|---|---|---|---|---|
| Treatment group | Total cholesterol (mg/dl) | | Comparison Initial vs After 3 months | Triglycerides (mg/dl) | | Comparison Initial vs After 3 months |
| | Initial | After 3 months | | Initial | After 3 months | |
| Control group (only dietary control) (N=48) | 216.84 ±8.97 | 201.72 ±6.82 | P<0.001 | 224.87 ±8.25 | 208.86 ±9.25 | P<0.001 |
| Treated Salacia chinensis along with dietary control (N=53) | 221.88 ±10.02 | 197.45 ±8.52 | P<0.001 | 230.26 ±8.25 | 213.87 ±9.26 | P<0.001 |
| Treated with Test formulation along with dietary control (N+61) | 227.43 ±10.55 | 182.25 ±7.34 | P<0.001 | 229.85 ±10.30 | 172.24 ±8.04 | P<0.001 |

When the organic extract of Coccinia indica, Hippophae rhamnoides, Salacia chinensis was orally administered the most effective result were obtained. A significant regulation of blood glucose levels, modification of abnormal lipid metabolism along with triglycerides and improvement in psychological complaints were recorded under influence of this new herbal formulation. A general feeling of well being and improvement in mental and physical stress of the subjects was noticed and thus the subject's work performance improved significantly as shown in Table 6.

**Table 6:**

| Effect of organic extract of Salacia chinensis and test formulation on clinical Symptomatology in Pre-diabetic cases | | | | | | | | | | |
|---|---|---|---|---|---|---|---|---|---|---|
| | *Clinical Symptomatology* (*Improvement in percent*) | | | | | | | | | |
| Treatment Group | Neuro logical complications | | Early fatigue | | Anxiety | | Depression | | feeling of well being | |
| | Before treatment | After treatment (*12 wks*) | Before treatment | After treatment (*12 wks*) | Before treatment | After treatment (*12 wks*) | Before treatment | After treatment (*12 wks*) | Before treatment | After treatment (*12 wks*) |
| Control group (N=48) | 23 (47.91 %) | 25 (52.08%) | 41 (85.54%) | 38 (79.16%) | 36 (75%) | 37 (77.08%) | 33 (68.75%) | 34 (70.83%) | 46 (95.83%) | 45 (93.75%) |
| Treated with Salacia chinensis (N=53) | 41 (77.35%) | 22 (41.50%) | 50 (94.33%) | 28 (52.83%) | 47 (88.67%) | 24 (45.28%) | 38 (71.69%) | 21 (39.62%) | 48 (90.56%) | 28 (52.83%) |
| Treated with test formulation (N=61) | 53 (86.88%) | 19 (31.14%) | 59 (96.72%) | 18 (29.50%) | 57 (93.44%) | 21 (34.42%) | 46 (75.40%) | 19 (31.11%) | 58 (95.08%) | 11 (18.03%) |

It was found that when the organic extract of Coccinia indica along with the organic extract of Hippophae rhamnoides was given orally in the prescribed doses continuously for long period the subject showing evidence of potential diabetes mellitus exhibited reduction in fasting and post prandial blood glucose levels. A decreased blood pressure levels were also recorded among those cases showing mild to moderate elevation of systolic and diastolic blood pressure. The formulation of the present invention regulates the blood glucose levels, blood pressure levels, and abnormal lipo-proteins including triglycerides. When the organic extract of Coccinia indica along with the organic extract of dry fruits of Hippophae rhamnoides and the organic extract of Salacia chinensis was administered in a specific determine effective doses better results were obtained when the values of blood glucose levels were measured, both fasting and post prandial blood glucose levels reduced and regulated in more effective manner than the earlier group of study. It has been concluded that the formulation has potentiality to prevent the pre-diabetics from entering in to type II diabetes.

### EXAMPLES:

Example 1: The organic extract of Salacia chinensis in the dose of 25 - 30 mg was administered did not show any change in blood glucose level among alloxan and streptozotocin induced experimental rats.
Example 2: When the organic extract of Salacia chinensis was administered in the dose of 70-100 mg to hyperglycaemic experimental rats, 40 percent of reduction in high blood glucose level was noticed.
Example 3: When the organic extract of leaves of Coccinia indica in the dose of 10-20 mg was introduced to alloxan and streptozotocin induced hyperglycaemic rats no hypoglycaemic activity could be noticed. The organic extract of the leaves in the dose of 60-80 mg was orally administered exerted beneficial effect on blood glucose level.
Example 4: When the organic extract of Salacia chinensis in the dose of 40-60 mg and Coccinia indica in the dose of 30-50 mg was given to hyperglycaemic rats a significant reduction in blood glucose level was noticed.
Example 5: In this experimental group the organic extract of Salacia chinensis in the dose of 50-75 mg along with Hippophae rhamnoides in the dose of 30-50 mg was given orally to experimentally induced hyperglycaemic rats a significant fall in blood glucose level was recorded.
Example 6: The organic extract of Salacia chinensis in the dose of 30-50 mg, Hippophae rhamnoides in the dose of 25-35 mg and Coccinia indica in the dose of 25-35 mg was given to experimental hyperglycaemic rats maximum beneficial effects were noticed on blood glucose level and also in regulating the kidney function
Example 7: In another group of clinical study when the organic extract of Salacia chinensis administered in the dose of 350 - 500 mg per day along with the organic extract of Hippophae rhamnoides in the dose of 250-350 mg per day improvement in neurological complaints were noticed along with hypoglycaemic effects.
Example 8: When the organic extract of Salacia chinensis in the dose of 400-500 mg per day along with the organic extract of Hippophae rhamnoides in the dose of 200-350 mg per day was introduced a significant decrease in blood pressure was noticed among the pre-diabetic cases. A general body immunity also enhanced due to reduced oxidative injury and modification in abnormal immune complex phenomena.
Example 9: When the organic extract of Coccinia indica in the dose of 250-300 mg per day was given along with the organic extract of Salacia chinensis in the dose of 400-550 mg per day, a better results of both fasting and post prandial glucose levels were noticed. A general feeling of well being was also reported by the diabetic patients.
Example 10: When the organic extract of Hippophae rhamnoides in the dose of 300-500 mg and Coccinia indica in the dose of 200-250 mg was given a general feeling of well being along with improvement in anxiety level were noticed in pre-diabetes cases and type II diabetes also.
Example 11: When the organic extract of Coccinia indica in the dose of 200-300 mg per day was given along with the organic extract of hippophae rhamnoides in the dose of 250-450 mg per day, a significant reduction in total cholesterol and triglycerides were noticed among the diabetes mellitus cases who showed hypercholesterolemia and hypertrigly ceridemia.
Example 12: The cases of pre-diabetes receiving the organic extract of Coccinia indica in the dose of 250-350 mg per day along with the organic extract of Salacia chinensis in the dose of 350-400 mg per day was introduced, significant reduction in Low-density lipoprotein cholesterol and mild increase in high density lipoprotein cholesterol was noticed along with decrease in blood glucose level.
Example 13: When the organic extract of Coccinia indica in the dose of 200-300 mg per day along with the organic extract of Hippophae rhamnoides in the dose of 250-400 mg per day, organic extract of Salacia chinensis in the dose of 400-550 mg per day was administered among diabetes mellitus cases significant improvement in fasting and post prandial blood glucose levels were observed along with regulation of TC, LDL-c and triglycerides. Subjects also reported a general feeling of well being and better mental performance.

## Claims

1. An herbal formulation for the prevention of endothelial dysfunction Diabetes Mellitus and Diabetic Micro-Vascular complications comprising 350-550 mg Salacia chinensis, 200-400 mg Coccinia indica, 200-400 mg Hippophae rhamnoides for every 1000 mg of said formulation and optionally additives in trace amounts, in this herbal formulation,
- Salacia chinensis is obtained by hydro-alcoholic extraction of root of Salacia chinensis through hot percolation method by soxhlet apparatus using water and alcohol as a solvent, the extraction being carried out continuously for 70 hours at boiling point of the solvent used;
- Coccinia indica is obtained by hydro-alcoholic extraction of plant Coccinia indica with a hydro-alcoholic solvent 70:30 at the temperature of 60 to 70°C, continuously for 70 hours ;
- Hippophae rhamnoides is obtained by hydro-alcoholic extraction of dried fruit of Hippophae rhamnoides with a hydro-alcoholic solvent 70:30 at the temperature of 60 to 70°C, continuously for 70 hours.

2. The herbal formulation according to claim 1 or 2 comprising 400-500 mg Salacia chinensis, 200-300 mg Coccinia indica and 250-400 mg Hippophae rhamnoides, for every 1000 mg of said formulation and optionally additives in trace amounts.

3. The herbal formulation as claimed in claims 1 to 3 wherein said additives are selected from mineral, vitamin, salts and binders.

4. The herbal formulation claimed in claim 1, 2 or 3 obtained by mixing an organic extract of Salacia chinensis , Hippophae rhamnoides and Coccinia indica and optionnaly adding additives in trace amounts.

5. Herbal formulation for the use for decreasing the blood pressure in pre-diabetic cases comprising 400-500 mg per day of the organic extract of Salacia chinensis and 200-350 mg per day of the organic extract of Hippophae rhamnoides, wherein in this herbal formulation,
- Salacia chinensis is obtained by hydro-alcoholic extraction of root of Salacia chinensis through hot percolation method by soxhlet apparatus using water and alcohol as a solvent, the extraction being carried out continuously for 70 hours at boiling point of the solvent used;
- Hippophae rhamnoides is obtained by hydro-alcoholic extraction of dried fruit of Hippophae rhamnoides with a hydro-alcoholic solvent 70:30 at the temperature of 60 to 70°C, continuously for 70 hours.

6. Herbal formulations for the use for the reduction of both fasting and post-prandial glucose levels in diabetic patients comprising 250-300 mg per day of the organic extract of Coccinia indica and 400-550 mg per day of the organic extract of Salacia chinensis, in this herbal formulation,
- Salacia chinensis is obtained by hydro-alcoholic extraction of root of Salacia chinensis through hot percolation method by soxhlet apparatus using water and alcohol as a solvent, the extraction being carried out continuously for 70 hours at boiling point of the solvent used;
- Coccinia indica is obtained by hydro-alcoholic extraction of plant Coccinia indica with a hydro-alcoholic solvent 70:30 at the temperature of 60 to 70°C, continuously for 70 hours.

7. Herbal formulations for the use for the reduction of total cholesterol and triglycerides in diabetes mellitus cases showing hypercholesterolemia and hypertriglyceridemia, comprising 200-300 mg per day of the organic extract of Coccinia indica and 250-450 mg per day of the organic extract of Hippophae rhamnoides, in this herbal formulation,
- Coccinia indica is obtained by hydro-alcoholic extraction of plant Coccinia indica with a hydro-alcoholic solvent 70:30 at the temperature of 60 to 70°C, continuously for 70 hours ;
- Hippophae rhamnoides is obtained by hydro-alcoholic extraction of dried fruit of Hippophae rhamnoides with a hydro-alcoholic solvent 70:30 at the temperature of 60 to 70°C, continuously for 70 hours.

8. Herbal formulation for the use for the reduction in low-density lipoprotein cholesterol and the increase in high density lipoprotein cholesterol and decrease of blood pressure, comprising 250-350 mg per day of the organic extract of Coccinia indica and 350-400 mg per day of the organic extract of Salacia chinensis, in this herbal formulation,
- Salacia chinensis is obtained by hydro-alcoholic extraction of root of Salacia chinensis through hot percolation method by soxhlet apparatus using water and alcohol as a solvent, the extraction being carried out continuously for 70 hours at boiling point of the solvent used;
- Coccinia indica is obtained by hydro-alcoholic extraction of plant Coccinia indica with a hydro-alcoholic solvent 70:30 at the temperature of 60 to 70°C, continuously for 70 hours.

9. Herbal formulation for the use for reduction of fasting and post prandial blood glucose levels and regulation of TC, LDL-c and tryglicerides in diabetes mellitus cases comprising 200-300 mg per day of the organic extract of Coccinia indica, 250-400 mg per day of the extract of Hippophae rhamnoides and 400-550 mg per day of the organic extract of Salacia chinensis, in this herbal formulation,
- Salacia chinensis is obtained by hydro-alcoholic extraction of root of Salacia chinensis through hot percolation method by soxhlet apparatus using water and alcohol as a solvent, the extraction being carried out continuously for 70 hours at boiling point of the solvent used;
- Coccinia indica is obtained by hydro-alcoholic extraction of plant Coccinia indica with a hydro-alcoholic solvent 70:30 at the temperature of 60 to 70°C, continuously for 70 hours ;
- Hippophae rhamnoides is obtained by hydro-alcoholic extraction of dried fruit of Hippophae rhamnoides with a hydro-alcoholic solvent 70:30 at the temperature of 60 to 70°C, continuously for 70 hours.

## Patentansprüche

1. Pflanzliche Zubereitung zur Vorbeugung von Diabetes mellitus mit endothelialer Dysfunktion und diabetischen, mikrovaskulären Komplikationen, enthaltend 350-550 mg Salacia chinensis, 200-400 mg Coccinia indica, 200-400 mg Hippophae rhamnoides auf jede 1000 mg besagter Zubereitung, und optional Spuren von Additiven, wobei in dieser pflanzlichen Zusammensetzung
- Salacia chinensis durch hydro-alkoholische Extraktion der Wurzel von Salacia chinensis per Heißperkulationsverfahren über eine Soxhlet-Vorrichtung unter Verwendung von Wasser und Alkohol als Lösungsmittel erhalten wird, wobei die Extraktion kontinuierlich über 70 Stunden bei der Siedetemperatur des verwendeten Lösungsmittels durchgeführt wird;
- Coccinia indica durch hydro-alkoholische Extraktion der Pflanze Coccinia indica mit einem 70:30 hydro-alkoholischen Lösungsmittel bei der Temperatur von 60 bis 70 °C, kontinuierlich über 70 Stunden, erhalten wird;
- Hippophae rhamnoides durch hydro-alkoholische Extraktion der getrockneten Früchte von Hippophae rhamnoides mit einem 70:30 hydro-alkoholischen Lösungsmittel bei der Temperatur von 60 bis 70 °C, kontinuierlich über 70 Stunden, erhalten wird.

2. Pflanzliche Zusammensetzung gemäß Anspruch 1 oder 2, enthaltend 400-500 mg Salacia chinensis, 200-300 mg Coccinia indica und 250-400 mg Hippophae rhamnoides auf jede 1000 mg besagter Zubereitung, und optional Spuren von Additiven.

3. Pflanzliche Zusammensetzung wie in den Ansprüchen 1 bis 3 beansprucht, wobei besagte Additive ausgewählt sind aus Mineral, Vitamin, Salze und Bindemittel.

4. Pflanzliche Zusammensetzung wie in Anspruch 1, 2 oder 3 beansprucht, erhalten durch Mischen eines organischen Extrakts von Salacia chinensis, Hippophae rhamnoides und Coccinia indica, und einer optionalen Zugabe von Spuren von Additiven.

5. Pflanzliche Zusammensetzung zur Verwendung in der Verringerung des Blutdrucks in prä-diabatischen Fällen, enthaltend 400-500 mg pro Tag von dem organischen Extrakt von Salacia chinensis und 200-350 mg pro Tag von dem organischen Extrakt von Hippophae rhamnoides, wobei in dieser pflanzlichen Zusammensetzung,
- Salacia chinensis durch hydro-alkoholische Extraktion der Wurzel von Salacia chinensis per Heißperkulationsverfahren über eine Soxhlet-Vorrichtung unter Verwendung von Wasser und Alkohol als Lösungsmittel erhalten wird, wobei die Extraktion kontinuierlich über 70 Stunden bei der Siedetemperatur des verwendeten Lösungsmittels durchgeführt wird;
- Hippophae rhamnoides durch hydro-alkoholische Extraktion der getrockneten Früchte von Hippophae rhamnoides mit einem 70:30 hydro-alkoholischen Lösungsmittel bei der Temperatur von 60 bis 70 °C, kontinuierlich über 70 Stunden, erhalten wird.

6. Pflanzliche Zusammensetzung zur Verwendung in der Verringerung sowohl der Hunger- als auch postprandialen Glukose-Werte in diabetischen Patienten, enthaltend 250-300 mg pro Tag von dem organischen Extrakt von Coccinia indica und 400-550 mg pro Tag von dem organischen Extrakt von Salacia chinensis, wobei in dieser pflanzlichen Zusammensetzung,
- Salacia chinensis durch hydro-alkoholische Extraktion der Wurzel von Salacia chinensis per Heißperkulationsverfahren über eine Soxhlet-Vorrichtung unter Verwendung von Wasser und Alkohol als Lösungsmittel erhalten wird, wobei die Extraktion kontinuierlich über 70 Stunden bei der Siedetemperatur des verwendeten Lösungsmittels durchgeführt wird;
- Coccinia indica durch hydro-alkoholische Extraktion der Pflanze Coccinia indica mit einem 70:30 hydro-alkoholischen Lösungsmittel bei der Temperatur von 60 bis 70 °C, kontinuierlich über 70 Stunden, erhalten wird.

7. Pflanzliche Zusammensetzung zur Verwendung in der Verringerung des Gesamtcholesterins und der Triglyceride in Diabetes mellitus Fällen, die eine Hypercholesterinämie und Hypertriglyceridämie zeigen, enthaltend 200-300 mg pro Tag von dem organischen Extrakt von Coccinia indica und 250-450 mg pro Tag von dem organischen Extrakt von Hippophae rhamnoides, wobei in dieser pflanzlichen Zusammensetzung,
- Coccinia indica durch hydro-alkoholische Extraktion der Pflanze Coccinia indica mit einem 70:30 hydro-alkoholischen Lösungsmittel bei der Temperatur von 60 bis 70 °C, kontinuierlich über 70 Stunden, erhalten wird;
- Hippophae rhamnoides durch hydro-alkoholische Extraktion der getrockneten Früchte von Hippophae rhamnoides mit einem 70:30 hydro-alkoholischen Lösungsmittel bei der Temperatur von 60 bis 70 °C, kontinuierlich über 70 Stunden, erhalten wird.

8. Pflanzliche Zusammensetzung zur Verwendung in der Verringerung des LDL-Cholesterins und der Erhöhung des HDL-Cholesterins und der Verringerung des Blutdrucks, enthaltend 250-350 mg pro Tag von dem organischen Extrakt von Coccinia indica und 350-400 mg pro Tag von dem organischen Extrakt von Salacia chinensis, wobei in dieser pflanzlichen Zusammensetzung,
- Salacia chinensis durch hydro-alkoholische Extraktion der Wurzel von Salacia chinensis per Heißperkulationsverfahren über eine Soxhlet-Vorrichtung unter Verwendung von Wasser und Alkohol als Lösungsmittel erhalten wird, wobei die Extraktion kontinuierlich über 70 Stunden bei der Siedetemperatur des verwendeten Lösungsmittels durchgeführt wird;
- Coccinia indica durch hydro-alkoholische Extraktion der Pflanze Coccinia indica mit einem 70:30 hydro-alkoholischen Lösungsmittel bei der Temperatur von 60 bis 70 °C, kontinuierlich über 70 Stunden, erhalten wird.

9. Pflanzliche Zusammensetzung zur Verwendung in der Reduktion der Hunger- und post-prandialen Blutglukose-Werte und der Regulation von TC, LDC-c und Triglyceride in Diabates mellitus Fällen, enthaltend 200-300 mg pro Tag von dem organischen Extrakt von Coccinia indica und 250-400 mg pro Tag von dem Extrakt von Hippophae rhamnoides und 400-550 mg pro Tag von dem organischen Extrakt von Salaica chinensis, wobei in dieser pflanzlichen Zusammensetzung,
- Salacia chinensis durch hydro-alkoholische Extraktion der Wurzel von Salacia chinensis per Heißperkulationsverfahren über eine Soxhlet-Vorrichtung unter Verwendung von Wasser und Alkohol als Lösungsmittel erhalten wird, wobei die Extraktion kontinuierlich über 70 Stunden bei der Siedetemperatur des verwendeten Lösungsmittels durchgeführt wird;
- Coccinia indica durch hydro-alkoholische Extraktion der Pflanze Coccinia indica mit einem 70:30 hydro-alkoholischen Lösungsmittel bei der Temperatur von 60 bis 70 °C, kontinuierlich über 70 Stunden, erhalten wird;
- Hippophae rhamnoides durch hydro-alkoholische Extraktion der getrockneten Früchte von Hippophae rhamnoides mit einem 70:30 hydro-alkoholischen Lösungsmittel bei der Temperatur von 60 bis 70 °C, kontinuierlich über 70 Stunden, erhalten wird.

## Revendications

1. Formulation à base de plantes pour la prévention du diabète sucré avec dysfonctionnement endothélial et des complications microvasculaires diabétiques comprenant de 350 à 550 mg de Salacia chinensis, de 200 à 400 mg de Coccinia indica, de 200 à 400 mg de Hippophae rhamnoides pour 1000 mg de ladite formulation et facultativement des additifs en quantités infimes, dans cette formulation à base de plante :
- Salacia chinensis est obtenue par extraction hydroalcoolique de racine de Salacia chinensis par le biais d'un procédé de percolation à chaud au moyen d'un appareil soxhlet en utilisant de l'eau et un alcool en tant que solvant, l'extraction étant réalisée de manière continue pendant 70 heures au point d'ébullition du solvant utilisé ;
- Coccinia indica est obtenue par extraction hydroalcoolique de la plante Coccinia indica avec un solvant hydroalcoolique 70:30 à la température de 60 à 70 °C, de manière continue pendant 70 heures ;
- Hippophae rhamnoides est obtenue par extraction hydroalcoolique du fruit séché de Hippophae rhamnoides avec un solvant hydroalcoolique 70:30 à la température de 60 à 70 °C, de manière continue pendant 70 heures.

2. Formulation à base de plantes selon la revendication 1 ou 2, comprenant de 400 à 500 mg de Salacia chinensis, de 200 à 300 mg de Coccinia indica et de 250 à 400 mg de Hippophae rhamnoides, pour 1000 mg de ladite formulation et facultativement des additifs en quantités infimes.

3. Formulation à base de plantes selon la revendication 1 ou 2, dans laquelle lesdits additifs sont choisis parmi un minéral, une vitamine, des sels et des liants.

4. Formulation à base de plantes selon la revendication 1, 2 ou 3, obtenue par mélange d'un extrait organique de Salacia chinensis, de Hippophae rhamnoides et de Coccinia indica et facultativement par ajout d'additifs en quantités infimes.

5. Formulation à base de plantes pour une utilisation afin de réduire la pression artérielle dans des cas de pré-diabète comprenant de 400 à 500 mg par jour de l'extrait organique de Salacia chinensis et de 200 à 350 mg par jour de l'extrait organique de Hippophae rhamnoides, dans cette formulation à base de plantes :
- Salacia chinensis est obtenue par extraction hydroalcoolique de racine de Salacia chinensis par le biais d'un procédé de percolation à chaud au moyen d'un appareil soxhlet en utilisant de l'eau et un alcool en tant que solvant, l'extraction étant réalisée de manière continue pendant 70 heures au point d'ébullition du solvant utilisé ;
- Hippophae rhamnoides est obtenue par extraction hydroalcoolique du fruit séché de Hippophae rhamnoides avec un solvant hydroalcoolique 70:30 à la température de 60 à 70 °C, de manière continue pendant 70 heures.

6. Formulation à base de plantes pour une utilisation afin de réduire à la fois la glycémie à jeun et la glycémie post-prandiale chez les patients diabétiques comprenant de 250 à 300 mg par jour de l'extrait organique de Coccinia indica et de 400 à 550 mg par jour de l'extrait organique de Salacia chinensis, dans cette formulation à base de plantes :
- Salacia chinensis est obtenue par extraction hydroalcoolique de racine de Salacia chinensis par le biais d'un procédé de percolation à chaud au moyen d'un appareil soxhlet en utilisant de l'eau et un alcool en tant que solvant, l'extraction étant réalisée de manière continue pendant 70 heures au point d'ébullition du solvant utilisé ;
- Coccinia indica est obtenue par extraction hydroalcoolique de la plante Coccinia indica avec un solvant hydroalcoolique 70:30 à la température de 60 à 70 °C, de manière continue pendant 70 heures.

7. Formulation à base de plantes pour une utilisation afin de réduire le cholestérol total et les triglycérides dans des cas de diabète sucré présentant une hypercholestérolémie et une hypertriglycéridémie comprenant de 200 à 300 mg par jour de l'extrait organique de Coccinia indica et de 250 à 450 mg par jour de l'extrait organique de Hippophae rhamnoides, dans cette formulation à base de plantes :
- Coccinia indica est obtenue par extraction hydroalcoolique de la plante Coccinia indica avec un solvant hydroalcoolique 70:30 à la température de 60 à 70 °C, de manière continue pendant 70 heures ;
- Hippophae rhamnoides est obtenue par extraction hydroalcoolique du fruit séché de Hippophae rhamnoides avec un solvant hydroalcoolique 70:30 à la température de 60 à 70 °C, de manière continue pendant 70 heures.

8. Formulation à base de plantes pour une utilisation afin de réduire le cholestérol LDL, afin d'augmenter le cholestérol HDL et afin de réduire la pression artérielle comprenant de 250 à 350 mg par jour de l'extrait organique de Coccinia indica et de 350 à 400 mg par jour de l'extrait organique de Salacia chinensis, dans cette formulation à base de plantes :
- Salacia chinensis est obtenue par extraction hydroalcoolique de racine de Salacia chinensis par le biais d'un procédé de percolation à chaud au moyen d'un appareil soxhlet en utilisant de l'eau et un alcool en tant que solvant, l'extraction étant réalisée de manière continue pendant 70 heures au point d'ébullition du solvant utilisé ;
- Coccinia indica est obtenue par extraction hydroalcoolique de la plante Coccinia indica avec un solvant hydroalcoolique 70:30 à la température de 60 à 70 °C, de manière continue pendant 70 heures.

9. Formulation à base de plantes pour une utilisation afin de réduire la glycémie à jeun et la glycémie post-prandiale et de réguler le cholestérol total, le cholestérol LDL et les triglycérides dans les cas de diabète sucré comprenant de 200 à 300 mg par jour de l'extrait organique de Coccinia indica, de 250 à 400 mg par jour de l'extrait organique de Hippophae rhamnoides et de 400 à 550 mg par jour de l'extrait organique de Salacia chinensis, dans cette formulation à base de plantes :
- Salacia chinensis est obtenue par extraction hydroalcoolique de racine de Salacia chinensis par le biais d'un procédé de percolation à chaud au moyen d'un appareil soxhlet en utilisant de l'eau et un alcool en tant que solvant, l'extraction étant réalisée de manière continue pendant 70 heures au point d'ébullition du solvant utilisé ;
- Coccinia indica est obtenue par extraction hydroalcoolique de la plante Coccinia indica avec un solvant hydroalcoolique 70:30 à la température de 60 à 70 °C, de manière continue pendant 70 heures ;
- Hippophae rhamnoides est obtenue par extraction hydroalcoolique du fruit séché de Hippophae rhamnoides avec un solvant hydroalcoolique 70:30 à la température de 60 à 70 °C, de manière continue pendant 70 heures.
